# EUROPÄISCHE PATENTSCHRIFT

(11) **EP 1 398 010 B1**
(45) Veröffentlichungstag und Bekanntmachung des Hinweises auf die Patenterteilung: **06.02.2008**
(21) Anmeldenummer: 03020444.0
(22) Anmeldetag: 12.09.2003
(51) Int. Cl.: A61F 5/02, A61F 5/058

(54) **Clavicula-Bandage**
Clavicle bandage
Bandage claviculaire

(30) Priorität: 14.09.2002 DE 10242771
(43) Veröffentlichungstag der Anmeldung: 17.03.2004
(73) Patentinhaber: Paul Hartmann Aktiengesellschaft, 89522 Heidenheim (DE)
(72) Erfinder: Gemerodt, Hans-Jürgen, 37581 Wanfried (DE)
(74) Vertreter: Langöhrig, Angelika Beate

(56) Entgegenhaltungen:
- EP-A- 0 781 536
- DE-U- 29 720 920
- FR-A- 2 610 516
- GB-A- 2 159 058
- US-A- 5 133 340
- US-A- 5 395 306
- US-A- 5 662 512

## Beschreibung

Die Erfindung betrifft eine Clavicula-Bandage mit zwei verstellbaren Schulterbändern, wobei die Schulterbänder mit einem jeweils ersten Ende an einem gemeinsamen, in einer Gebrauchslage auf einem Rücken eines Patienten zu liegenden kommenden Halteelement fixiert sind und zweite Enden der Bänder durch Ausnehmungen im Halteelement durchgezogen sind.

Derartige Clavicula-Bandagen werden vielfach zur Behandlung von Verletzungen, insbesondere Frakturen des Schlüsselbeins, verwendet.

Frakturen des Schlüsselbeins und andere Verletzungen desselben werden zum überwiegenden Teil durch Bandagen oder Verbände behandelt, wobei hierbei insbesondere der "Rucksackverband" bekannt ist, bei dem beispielsweise ein Trikotschlauch mit Watte gefüllt und auf passende Länge zugeschnitten wird. Dieser gefüllte Schlauch wird im Nackenbereich des Patienten angelegt, über beide Schlüsselbeine und Achselhöhlen geführt und auf dem Rücken verknotet.

Nachteilig bei dieser Art der Bandage ist, dass die Herstellung in der Klinik selbst erfolgen muss, was sehr zeitintensiv ist. Des weiteren ist der Verband verhältnismäßig dehnfähig, so dass er sich unter Zug längt, so dass der Verband mehrmals nachgestellt werden muss. Schließlich besteht die Gefahr, dass aufgrund des Knotens im Rückenbereich Reizungen oder gar Verletzungen entstehen und der Verband im Nackenbereich drückt oder gar verrutscht.

In Weiterbildung derartiger Verbände sind aus der EP 0 379 929 A1 Clavicula-Bandagen bekannt, die aus zwei in ihrer Länge einstellbaren Gurten gebildet sind. Die Gurte weisen an ihren freien Enden Mittel zur Schlaufenbildung auf. Die Gurte sind mit ihrem anderen Ende an einem Ring, der vorzugsweise mit einem flachen Querschnitt ausgeführt ist, unabhängig voneinander befestigt. Weiterhin bestehen die Gurte aus im wesentlichen nicht dehnfähigem Material.

Diese Ausgestaltung besitzt jedoch den Nachteil, dass die Gurte vom Rücken kommend meist schlecht oder ungenau positioniert sind und im angelegten Zustand zunehmend nach lateral wandern, was die Funktion und den Tragekomfort wesentlich verschlechtert. Der Ring führt im Bereich der medialen Schulterblattgräten zu Druckstellen und durch teilweise Verformung zu einem Verlust der Stabilität der Bandage.

Darüber hinaus besteht insbesondere der Nachteil, dass die Bandage nicht durch den Patienten selber angelegt werden kann, da die Schlaufenbildung und damit der Verschluss und das Spannen der Bandage im Bereich des Patientenrückens erfolgt und damit stets eine zweite Person notwendig ist.

Desweiteren ist beispielsweise aus der EP 1 077 050 A1 eine Clavicula-Bandage bekannt, wobei hier die beiden ersten Enden, die am oberen Ende in der Gebrauchslage des Ringes befestigt sind, durch eine Stofftasche in ihrer Lage zueinander fixiert sind, so dass die Position der Bänder im wesentlichen fest ist und ein Verrutschen nach lateral verhindert werden kann. Darüber hinaus soll der Ring einen flachen und elliptischen Querschnitt aufweisen. Genau wie in der zuvor beschriebenen Schrift erfolgt das Fixieren der Bandage im Rückenbereich des Patienten durch Klettverschlüsse, wobei die freien Enden jeweils auf sich selber festgelegt werden. Es ist daher notwendig, dass eine zweite Person zum Anlegen der Bandage zur Verfügung steht.

Des weiteren zeigt DE 297 20 920 U1 eine Clavicula-Bandage mit einem ringförmigen Befestigungselement, das vorzugsweise in Form eines gleichschenkligen Dreiecks ausgebildet ist, wobei die ersten Enden durch einen im wesentlichen waagrecht verlaufenden Schenkel in der Gebrauchsposition hindurchgezogen werden und miteinander verbunden sind und die freien Enden durch die beiden weiteren Seiten des gleichschenkligen Dreiecks hindurchgezogen und auf sich selbst festgelegt werden. Auch hier besteht wiederum der bekannte Nachteil, dass die Bandagen nur durch eine zweite Person angelegt und befestigt werden können.

Eine weitere Ausgestaltung offenbart DE 195 05 854 C2, die eine Clavicula-Bandage offenbart, die ein längliches Halteelement aufweist, das im Gebrauchsfall auf dem Rücken des Patienten zu liegen kommt, und wobei jeweils die ersten Enden der beiden Schulterbänder im oberen Bereich des gemeinsamen Halteelements dauerhaft fixiert und mit ihrem jeweils anderen Ende im unteren Bereich des Halteelements mittels zweier Schnallen lösbar so festgelegt sind, dass die Schnallen um einen Drehpunkt verschwenkbar sind. Darüber hinaus ist eine Höhenverstellbarkeit der Schulterbänder an dem Halteelement vorgesehen. Auch hier besteht der Nachteil, dass zum Anlegen und Verstellen der Clavicula-Bandage stets eine zweite Person notwendig ist.

Des weiteren offenbart DE 42 11 023 C1 eine einteilige Bandage für ein Schlüsselbein mit einem den Rücken quer überspannenden Rückengurt, an den sich unter den Achsenhöhlen verlaufende, über die Schlüsselbeine erstreckende Schultergurte anschließen, die sich im Bereich der Mitte des Rückengurts an einer Kreuzungsstelle kreuzen und im daran anschließenden Endteil über die Hüften bis zum Bauch auslaufen, wo die Endteile mittels eines Klettverschlusses miteinander verbunden werden.

Diese Ausgestaltung einer Clavicula-Bandage besitzt den Nachteil einer sehr aufwendigen Konfiguration, bei der die Bänder im Rücken über Kreuz geführt werden. Eine Zugverteilung, wie sie im Stand der Technik mittels des Halteelementes vorgesehen ist, offenbart die Schrift nicht. Darüber hinaus besteht insbesondere aufgrund des fehlenden Halteelementes die Gefahr, dass sich die Zugspannung löst und ein häufiges Nachstellen der Bandage erforderlich ist.

Schließlich besteht bei dieser Bandage ähnlich wie beim "Rucksack"-Verband die Gefahr des lateralen Verrutschens.

Eine Clavicula-Bandage gemäß dem Oberbegriff von Anspruch 1 ist aus der EP-A-0 781 536 bekannt.

Vor dem aufgezeigten Hintergrund ist es die Aufgabe der vorliegenden Erfindung, die eingangs erwähnte Clavicula-Bandage dahingehend zu verbessern, dass sie eine hohe Therapiesicherheit gewährleistet, d. h. einen anatomisch guten Sitz ohne Verrutschen und Lockern und dabei einfach anzulegen und für den Patienten angenehm zu tragen ist, sowie ein Anlegen durch den Patienten selber ermöglicht.

Die Erfindung löst diese Aufgabe durch eine Clavicula-Bandage gemäß Anspruch 1.

Die erfindungsgemäße Bandage weist daher den Vorteil auf, zum einen den Druck gleichmäßig über das Halteelement auf die Schlüsselbeine zu verteilen und eine sichere Fixation der Schulterbänder am Patienten sicherzustellen. Ein Verrutschen und damit auch Nachlassen bzw. Verändern der Zugkräfte auf das Schlüsselbein kann somit weitestgehend verhindert werden. Die Therapiesicherheit der beschriebenen Bandage ist daher verhältnismäßig hoch. Darüber hinaus besteht bei der erfindungsgemäßen Bandage der Vorteil, da die freien Enden so lang ausgebildet sind, dass sie durch das Halteelement durchgezogen und zur Bildung von zwei Schlaufen, die um die Schultern herumgelegt werden, dienen und gleichzeitig so lang sind, dass sie wieder vor der Brust eines Patienten zusammengeführt werden können, dass ein Nachspannen der Bandage einfach durch den Patienten selbst möglich ist. Insbesondere ist auch ein Anlegen durch den Patienten selber möglich, was bei einem Verschluss im Bereich des Rückens nur schwierig zu bewerkstelligen ist. Eine zweite Person, die die Bandage am Patienten anlegt, ist daher nicht notwendig. Durch das Halteelement wird der Zug auf die einzelnen Schulterbänder gleichzeitig auf den gesamten Bandagenapparat übertragen, und so ein möglichst gleichmäßiger Zug auf die Schlüsselbeine erzielt.

Nach einem ersten Ausführungsbeispiel kann vorgesehen sein, dass wenigstens ein freies Ende eines Schulterverbandes ein Klettverschlusselement aufweist, insbesondere ein Hakenelement, das mit einem korrespondierenden Klettverschlusselement oder dem Material der Schulterbänder des anderen freien Endes zusammenwirkt. So kann das Außenmaterial der Bandage selbst so gestaltet sein, dass die Häkchen eines Klettverschlusses auf ihm haften können. Die Klettverschlusselemente sind dabei so lang ausgestaltet, dass zum einen ein sicherer Halt auch bei den insbesondere bei Bewegung der Patienten auftretenden Zugkräften erzielt wird und die Bandage möglichst universell an die verschiedenen Körpergrößen von Patienten angepasst werden kann.

Die Schlaufen, die durch die Schulterbänder gebildet werden, verlaufen hierbei von oben um die Schultern herum ausgehend vom Halteelement zurück zum Halteelement und dann um den Bauch bzw. die Brust eines Patienten herum. Durch Zug an den freien Enden der Schulterbänder, die vor der Brust eines Patienten miteinander verbindbar sind, können die um die Schultern liegenden Schlaufen der Schulterbänder in ihrer Länge eingestellt werden.

Das Halteelement kann dabei eine polygonale, insbesondere längliche Form mit insbesondere abgerundeten Ecken aufweisen. Insbesondere kann es sich hierbei um eine sechseckige Form handeln, wobei ein erster rechteckiger Teil vorgesehen ist und ein zweiter trapezförmiger Teil, der an den rechteckigen Teil in Richtung nach unten (kaudal) in der Gebrauchslage anschließt, wobei die Kürzere der parallelen Seiten nach unten weist und die Breitere der Seitenlänge des rechteckigen Teils entspricht, an der sie anschließt.

Die Längsachse des länglichen Halteelementes kann sich dabei in Richtung der Längsachse eines die Bandage tragenden Patienten erstrecken.

Die Winkel des trapezförmigen Teiles des Halteelementes, wobei der trapezförmige und der rechteckige Teil einstückig miteinander verbunden sein können, sind dabei so gestaltet, dass ein möglichst günstiger Winkel zum Spannen und gleichzeitig zum Herumführen nach vorne der freien Enden gegeben ist.

Insbesondere kann vorgesehen sein, dass in dem Halteelement drei Schlitze vorgesehen sind, wobei ein erster Schlitz zur Festlegung der beiden ersten Enden der Schulterbänder quer zur Längsachse des Halteelementes verläuft und im Bereich der in einer Gebrauchslage nach oben weisenden Kante angeordnet ist, insbesondere des rechteckigen Bestandteils des Halteelementes. Weiterhin können die beiden anderen Schlitze am unteren Ende in der Gebrauchslage des Halteelementes liegen, wobei die Schlitze symmetrisch zur Längsachse verlaufen und einen Winkel von 10 bis 45° mit der Längsachse vom unteren Ende des Halteelementes her einschließen. Insbesondere kann die trapezförmige Form an die Form der Schlitzungen angepasst sein, so dass insbesondere die nicht parallelen Seiten des Trapezes parallel zu den Schlitzen verlaufen.

Weiterhin kann vorgesehen sein, dass die Schulterbänder aus einem Weichschaumverbundstoff, insbesondere mit einem vom Patienten abgewandten Obermaterial aus Polyamidvelours, einem Untermaterial aus Baumwoll-Jersey und einem Polyesterschaumstoff bestehen.

Das Halteelement kann dabei aus Hartpolyethylen hergestellt sein, um allergische Reaktionen auf die Haut zu minimieren. Es ist dabei so gestaltet, dass ein Kanten- oder Randdruck des Halteelementes auf den Rücken des Patienten vermieden wird und eine Verformung des Halteelementes auch im angelegten und unter Zug stehenden Zustand nicht zu beobachten ist.

Erfindungsgemäß ist das Halteelement als Zugverteilerplatte ausgebildet, um die Bewegungsfreiheit des Patienten zum einen möglichst wenig zu beeinflussen und zu beschränken und auf der anderen Seite die Frakturstellen effektiv ruhigzustellen. Auf diese Weise kann eine patientenindividuelle Einstellung erfolgen, um eine erfolgreiche Therapie durchführen zu können.

Es kann weiterhin vorgesehen sein, dass die ersten Enden der Schulterbänder durch eine Stofftasche bezüglich ihrer Lage zueinander fixiert sind, wobei die Stofftasche insbesondere die Form eines nicht regelmäßigen Pentagons aufweisen kann, so dass die vom Halteelement abgehenden Schulterbänder zwischen sich einen Winkel von 45 bis 95° einschließen. Die Stofftasche wird dabei aus einem Stoffabschnitt gebildet, der durch den korrespondierenden Schlitz geführt und dann umgeschlagen und aufeinander vernäht wird, wobei die Fixierung der Bänder durch Einlegen in die Stofftasche und Vernähen der Stofftasche mit denselben erfolgt. Durch die Ausbildung einer solchen Tasche kann bei der angelegten Bandage eine verbesserte Gurtführung erzielt werden, wobei die Spitze des Pentagons nach kranial weist.

Die Befestigung der ersten Enden mittels der Stofftasche wirkt einer Lateralisierung der Bänder im frontalen Bereich entgegen. Darüber hinaus wird auf diese Weise das Halteelement im oberen Bereich weiter gepolstert.

Das Innenmaterial bzw. Untermaterial aus Baumwoll-Jersey kann alternativ auch aus einem anderen schweißaufsaugenden Material, wie beispielsweise Polyamid, Polyester oder Baumwolle sowie Mischungen hieraus, gewählt werden. Die Polsterung, die beispielsweise aus Polyesterschaumstoff bestehen kann, kann beispielsweise so gewählt sein, dass eine ausreichende Sicherung gegen Druck erzielt wird.

Das Obermaterial ist dabei so zu wählen, dass eine weiche, insbesondere flauschige, Oberfläche, erzielt wird, auf der die Klettverschlüsse gut haften können. Darüber hinaus sind sämtliche Materialien möglichst luftdurchlässig ausgeführt, um die Gefahr eines Hitzestaus und der Schweißbildung zu verringern.

Weitere Vorteile und Merkmale der erfindungsgemäßen Clavicula-Bandage ergeben sich aus den weiteren Unterlagen. Die Erfindung wird im Folgenden anhand einer Zeichnung näher erläutert:

Dabei zeigen:

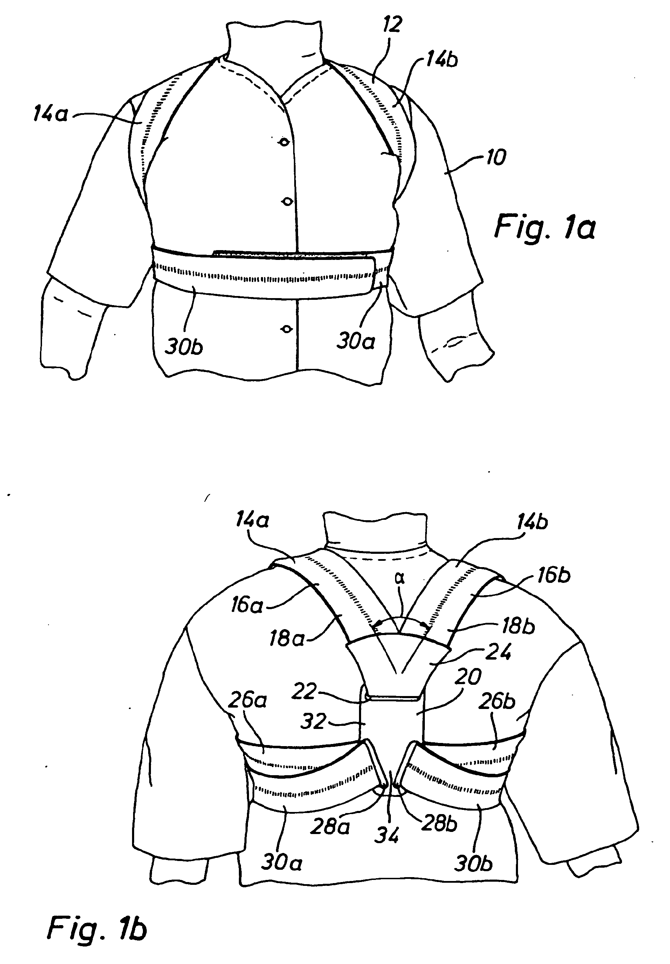

- Figur 1a: eine Vorderansicht einer erfindungsgemäßen Clavicula-Bandage in Gebrauchslage und
- Figur 1b: eine Rückenansicht von Figur 1a.

In Figur 1a ist ein Patient 10 gezeigt, der an seinem Oberkörper eine erfindungsgemäße Clavicula-Bandage 12 trägt. Die Bandage umfasst zwei Schlaufen 14a und 14b, die die Schultern des Patienten 10 umschlingen. Dadurch wird ein Schlüsselbein des Patienten 10 nach einer Fraktur stabilisiert.
Figur 1b zeigt nun eine Rückenansicht, wobei hier die Schlaufen 14a und 14b gesehen werden können, die dadurch gebildet werden, dass eine Clavicula-Bandage mit zwei verstellbaren Schulterbändern 16a und 16b, die jeweils mit einem ersten Ende 18a und 18b an einem Halteelement 20 festgelegt sind über die Schultern eines Patienten nach vorne geführt werden (Figur 1a) und dort unter den Armen hindurch verlaufend wieder zum Rücken (Figur 1b) geschlungen sind.

Zur Festlegung der ersten Enden 18a, 18b der Schulterbänder 16a, 16b weist das Halteelement 20, das eine längliche in Längsachse des Patienten verlaufende Form aufweist, an seinem oberen nach kranial weisenden Ende einen ersten quer zu seiner Längsachse verlaufenden Schlitz 22 auf, in dem die Schulterbänder 16a, 16b mit ihren ersten Enden 18a, 18b über eine Stofftasche 24 festgelegt sind, indem die Schulterbänder mit einem definierten Winkel (α) zueinander in der Stofftasche angeordnet und hiermit vernäht sind, wobei die Stofftasche die Form eines unregelmäßigen Pentagons aufweist, dessen Spitze nach kranial weist und die Schulterbänder derart angeordnet sind, dass sie einen Winkel zwischen 45 und 95° zwischen sich einschließen, wodurch die Schulterbänder 16a und 16b lateral gegen Verrutschen im vorderen Brustbereich des Patienten gesichert sind. Darüber hinaus dient die Stofftasche 24 zur Abpolsterung der Befestigungsstellen des Halteelements 20.

Die unter den Armen zurückgeführten Teile der Schlaufen 14a und 14b, nämlich 26a und 26b, die durch die freien Enden der Bandage gebildet werden, werden durch weitere Schlitze 28a und 28b des Halteelementes 20 hindurchgezogen, wobei die freien Enden 30a und 30b dann wiederum um den Patienten herum nach vorne in den Brustbereich geführt werden und dort über einen Klettverschluss miteinander verbunden werden können, wobei das freie Ende 30b ein Hakenelement eines Klettverschlusses aufweist, der mit dem Obermaterial des freien Endes 30a korrespondierend und verankernd zusammenwirkt.

Das Halteelement 20 ist dabei sechseckig gestaltet mit abgerundeten Kanten und besteht im wesentlichen aus einem rechteckigen Bestandteil 32 sowie einem trapezförmigen Bestandteil 34, die einstückig miteinander verbunden sind, wobei der rechteckige Bestandteil kranial und der trapezförmige Bestandteil kaudal angeordnet ist und die Schmalseite des trapezförmigen Bestandteiles 34 nach kaudal weist. Die Schlitze 28a und 28b sind dabei derart angeordnet, dass sie einen Winkel zwischen sich und der Längsachse des Halteelementes von 10 bis 45° einschließen und parallel zur Außenkante des trapezförmigen Bestandteiles 34 verlaufen.

Durch eine solche Gestaltung des Halteelementes 20 wird die Bewegungsfreiheit des Patienten am wenigsten eingeschränkt, und es entsteht auch nur eine geringe Druckbelastung auf die Wirbelsäule durch die Randbereiche des Halteelementes 20. Durch die nach vorne herumgeführten freien Enden 30a und 30b können die Schulterbänder 16a und 16b bezüglich der gebildeten Schlaufen 14a und 14b nachgespannt bzw. gespannt werden, so dass ein Anlegen durch den Patienten 10 alleine, ohne Hilfe einer weiteren Person, möglich ist. Dabei können die Schlaufen 14a, 14b bereits vor Anlegen gebildet werden. Durch das Hindurchziehen der freien Enden 30a und 30b durch die Schlitze 28a und 28b wird verhindert, aufgrund der bestehenden Reibungskräfte, dass sich die Bandage von selbst zu leicht lockert.

Durch die erfindungsgemäße Bandage kann ein guter Tragekomfort bei gleichzeitig einfacher Anlegbarkeit durch den Patienten selbst oder weitere Hilfspersonen sichergestellt werden, wobei das Halteelement als Zugteilerplatte dient und den Zug, der durch die freien Enden 30a und 30b in die Schulterbänder 16a und 16b eingeleitet wird, gleichmäßig auf beide Schultern und damit auf die Clavicula verteilt. Eine Therapie eines frakturierten Schlüsselbeins kann auf diese Weise besonders einfach erfolgen.

Die Bandage selbst besteht dabei aus einem Weichschaumverbundstoff, wobei die Oberseite aus einem Polyamidvelours mit einem Flächengewicht von 120 bis 125 g/m² besteht und der Anteil des Obermaterials am Weichschaumverbundstoff ca. 32 % beträgt.

Die Unterseite ist aus einem Baumwoll-Single-Jersey-Gewebe hergestellt mit einem Flächengewicht von 95 bis 100 g/m², wobei der Anteil des Materials der Unterseite ca. 26 % am Weichschaumverbundstoff ausmacht. Das Material ist dann mit einem Schaumstoff, nämlich einem Polyesterschaumstoff, gefüllt. Das Gesamtgewicht des Weichschaumverbundstoffes beträgt ca. 375 g/m².

Die einzelnen Schulterbänder 16a und 16b können dabei eine Breite von ca. 5 cm aufweisen. Die Dicke des Polsterstoffes beträgt ca. 1 cm im nicht komprimierten Zustand. Die Länge jedes der Schulterbänder kann ca. 130 cm betragen. Die Bandage ist auf diese Weise individuell an den Patienten anpassbar.

## Patentansprüche

1. Clavicula-Bandage mit zwei verstellbaren Schulterbändern, wobei die Schulterbänder (16a, b) mit einem jeweils ersten Ende (18a, b) an einem gemeinsamen auf einem Rücken eines Patienten zu liegen kommenden Halteelement (20) fixiert sind und zweite Enden (30a, b) der Schulterbänder (16a, b) durch Ausnehmungen (28a, b) im Halteelement (20) durchgezogen sind, zur Bildung von zwei, die Schultern des Patienten (10) umschließenden, längenverstellbaren Schlaufen (14a, b), **dadurch gekennzeichnet, dass** die freien Enden (30a, b) der Schulterbänder (16a, b) vor der Brust des Patienten (10) miteinander verbindbar sind und das Halteelement (20) als Zugverteilerplatte ausgebildet ist.

2. Clavicula-Bandage nach Anspruch 1, wobei wenigstens ein freies Ende (30b) eines Schulterbandes (16a) ein Klettverschlusselement aufweist, das mit einem korrespondierenden Klettverschlusselement oder dem Material der Schulterbänder (16a, b) selbst des anderen freien Endes (30a) zusammenwirkt.

3. Clavicula-Bandage nach Anspruch 1 oder 2, wobei die durch die Schulterbänder (16a, b) gebildeten Schlaufen (14a, b) durch Zug an den freien Enden (30a, b) der Schulterbänder (16a, b) verstellbar sind.

4. Clavicula-Bandage nach Anspruch 1 bis 3, wobei das Halteelement (20) eine polygonale, insbesondere längliche Form mit insbesondere abgerundeten Ecken aufweist, wobei sich die Längsachse des Haltelementes (20) in Richtung der Längsachse des die Bandage tragenden Patienten (10) erstreckt.

5. Clavicula-Bandage nach einem der vorangehenden Ansprüche, wobei in dem Halteelement (20) drei Schlitze (22, 28a, b) vorgesehen sind, wobei ein erster Schlitz (22) zur Festlegung der beiden ersten Enden (18a, b) der Schulterbänder (16a, b) quer zur Längsachse des Halteelementes (20) verläuft und im Bereich der in einer Gebrauchslage nach oben weisenden Kante angeordnet ist und wobei die anderen beiden Schlitze (28a, b) am in der Gebrauchslage unteren Ende des Halteelementes (20) liegen, wobei diese Schlitze (28a, b) symmetrisch zur Längsachse des Halteelementes (20) verlaufen und einen Winkel von 10°- 45° mit der Längsachse vom unteren Ende des Halteelementes (20) her einschließen.

6. Clavicula-Bandage nach Anspruch 5, wobei das Halteelement (20) bezüglich seiner äußeren Form an den Verlauf der Schlitze (22, 28a, b) angepasst ist.

7. Clavicula-Bandage nach einem der vorangehenden Ansprüche, wobei die Schulterbänder (16a, b) aus Weichschaumverbundstoff, insbesondere mit einem vom Patienten abgewandten Obermaterial aus Polyamid-Velours, einem Untermaterial aus Baumwoll-Jersey und einem Polyesterschaumstoff bestehen.

8. Clavicula-Bandage nach einem der vorangehenden Ansprüche, wobei das Halteelement (20) aus Hartpolyethylen besteht.

9. Clavicula-Bandage nach einem der vorangehenden Ansprüche, wobei die ersten Enden (18a, b) der Schulterbänder (16a, b) durch eine Stofftasche (24) bezüglich ihrer Lage zueiander fixiert sind, wobei die Stofftasche (24) insbesondere die Form eines nicht regelmäßigen Pentagons aufweist, so dass die vom Halteelement (20) abgehenden Schulterbänder (16a, b) zwischen sich einen Winkel von 45° bis 95° einschließen.

## Claims

1. Clavicle bandage with two adjustable shoulder straps, wherein the shoulder straps (16a, b) are each fixed by a first end (18a, b) to a common holding element (20), which is to rest on the back of a patient, and second ends (30a, b) of the shoulder straps (16a, b) are passed through openings (28a, b) in the holding element (20) in order to form two loops (14a, b), which surround the shoulders of the patient (10) and are adjustable in length, **characterised in that** the free ends (30a, b) of the shoulder straps (16a, b) can be connected to one another in front of the chest of the patient (10) and **in that** the holding element (20) is formed as a tension-distributing plate.

2. Clavicle bandage according to claim 1, wherein at least one free end (30b) of one shoulder strap (16a) has a hook-and-loop fastening element, which interacts with a corresponding hook-and-loop fastening element, or with the material of the shoulder strap (16a, b) itself, at the other free end (30a).

3. Clavicle bandage according to either claim 1 or claim 2, wherein the loops (14a, b) formed by the shoulder straps (16a, b) can be adjusted by pulling on the free ends (30a, b) of the shoulder straps (16a, b).

4. Clavicle bandage according to claims 1 to 3, wherein the holding element (20) has a polygonal shape, especially an oblong shape, in particular with rounded corners, the longitudinal axis of the holding element (20) extending in the direction of the longitudinal axis of the patient (10) wearing the bandage.

5. Clavicle bandage according to any one of the preceding claims, wherein three slots (22, 28a, b) are provided in the holding element (20), a first slot (22), for the fastening of the two first ends (18a, b) of the shoulder straps (16a, b), extending transversely to the longitudinal axis of the holding element (20) and being arranged in the region of the edge directed upwards during use, and both the other slots (28a, b) being disposed at the end of the holding element (20) pointing downwards during use, these slots (28a, b) extending symmetrically about the longitudinal axis of the holding element (20) and being at an angle of 10° - 45° to the longitudinal axis from the lower end of the holding element (20).

6. Clavicle bandage according to claim 5, wherein the external shape of the holding element (20) is adapted to the course of the slots (22, 28a, b).

7. Clavicle bandage according to any one of the preceding claims, wherein the shoulder straps (16a, b) consist of a flexible foam composite, in particular with an upper material of polyamide velour facing away from the patient, a lower material of cotton jersey, and a polyester foam.

8. Clavicle bandage according to any one of the preceding claims, wherein the holding element (20) consists of high-density polyethylene.

9. Clavicle bandage according to any one of the preceding claims, wherein the first ends (18a, b) of the shoulder straps (16a, b) are fixed in position relative to one another by a cloth pocket (24), said cloth pocket (24) being, in particular, in the shape of an irregular pentagon, such that the shoulder straps (16a, b) leading out of the holding element (20) are at an angle of 45° - 95° to one another.

## Revendications

1. Bandage claviculaire comportant deux bandes d'épaules réglables, les bandes d'épaules (16a, b) étant fixées avec une première extrémité (18a, b) à un élément de retenue (20) commun venant s'appliquer sur le dos d'un patient et les secondes extrémités (30a, b) des bandes d'épaules (16a, b) étant passées à travers des ouvertures (28a, b) pratiquées dans l'élément de retenue (20), afin de former deux boucles (14a, b) réglables en longueur entourant les épaules du patient (10), **caractérisé en ce que** les extrémités libres (30a, b) des bandes d'épaules (16a, b) peuvent être reliées entre elles devant la poitrine du patient (10) et **en ce que** l'élément de retenue (20) est réalisé sous la forme d'une plaque de répartition de traction.

2. Bandage claviculaire selon la revendication 1, au moins une extrémité libre (30b) d'une bande d'épaule (16a) présentant un élément de fermeture auto-agrippante qui coopère avec un élément de fermeture auto-agrippante correspondant, ou avec la matière même des bandes d'épaules (16a, b), de l'autre extrémité libre (30a).

3. Bandage claviculaire selon la revendication 1 ou 2, les boucles (14a, b) formées par les bandes d'épaules (16a, b) pouvant être réglées en tirant sur les extrémités libres (30a, b) des bandes d'épaules (16a, b).

4. Bandage claviculaire selon les revendications 1 à 3, l'élément de retenue (20) possédant une forme polygonale, en particulier oblongue, notamment avec des angles arrondis, l'axe longitudinal de l'élément de retenue (20) s'étendant dans le sens de l'axe longitudinal du patient (10) portant le bandage.

5. Bandage claviculaire selon l'une des revendications précédentes, trois fentes (22, 28a, b) étant prévues dans l'élément de retenue (20), une première fente (22), destinée à la fixation des deux premières extrémités (18a, b) des bandes d'épaules (16a, b), s'étendant transversalement à l'axe longitudinal de l'élément de retenue (20) et étant agencée dans la zone du bord orienté vers le haut dans une position d'utilisation, et les deux autres fentes (28a, b) étant situées au niveau de l'extrémité inférieure de l'élément de retenue (20) dans la position d'utilisation, ces fentes (28a, b) s'étendant de manière symétrique par rapport à l'axe longitudinal de l'élément de retenue (20) et formant un angle de 10° à 45° avec l'axe longitudinal depuis l'extrémité inférieure de l'élément de retenue (20).

6. Bandage claviculaire selon la revendication 5, l'élément de retenue (20) étant adapté, en ce qui concerne sa forme extérieure, au tracé des fentes (22, 28a, b).

7. Bandage claviculaire selon l'une des revendications précédentes, les bandes d'épaules (16a, b) étant en matière composite formant une mousse souple, notamment composée d'une matière superficielle, orientée à l'opposé du patient, en velours polyamide, d'une matière de doublure en jersey de coton et d'une mousse polyester.

8. Bandage claviculaire selon l'une des revendications précédentes, l'élément de retenue (20) étant en polyéthylène dur.

9. Bandage claviculaire selon l'une des revendications précédentes, les premières extrémités (18a, b) des bandes d'épaules (16a, b) étant fixées, quant à leur position l'une par rapport à l'autre, par une poche en tissu (24), la poche en tissu (24) présentant en particulier la forme d'un pentagone irrégulier de sorte que les bandes d'épaules (16a, b) sortant de l'élément de retenue (20) forment entre elles un angle de 45° à 95°.
